# EUROPÄISCHE PATENTANMELDUNG

(11) **EP 2 359 799 A1**
(43) Veröffentlichungstag der Anmeldung: **24.08.2011**
(21) Anmeldenummer: 10153721.5
(22) Anmeldetag: 16.02.2010
(51) Int. Cl.: A61J 1/14, B65D 51/22, A61M 39/26

(54) **Verschlusskappe für ein Behältnis zur Aufnahme von medizinischen Flüssigkeiten und Behältnis**

(71) Anmelder: Fresenius Kabi Deutschland GmbH, 61352 Bad Homburg (DE)
(72) Erfinder: Brandenburger, Torsten, 61203, Reichelsheim (DE); Rahimy, Ismael, 61169, Friedberg (DE)
(74) Vertreter: Richly, Erik

(57) **Zusammenfassung**

Die Erfindung betrifft eine Verschlusskappe für ein Behältnis zur Aufnahme von medizinischen Flüssigkeiten und ein Behältnis für medizinische Flüssigkeiten, dass eine derartige Verschlusskappe aufweist.

Die erfindungsgemäße Verschlusskappe (1) umfasst einen ersten Anschluss (2) zum nadelfreien Zuspritzen einer medizinischen Flüssigkeit und einen zweiten Anschluss (3) zum nadelfreien Entnehmen einer medizinischen Flüssigkeit, wobei der erste Anschluss (2) ein nach außen weisendes erstes Anschlussteil (4) mit einer konischen Ausnehmung (8) zur abdichtenden Aufnahme eines Kegelschaftes (26) einer anzuschließenden ersten Vorrichtung (28, 29) und der zweite Anschluss (3) ein nach außen weisendes zweites Anschlussteil (5) mit einer konischen Ausnehmung (9) zur abdichtenden Aufnahme eines Kegelschaftes einer anzuschließenden zweiten Vorrichtung aufweist.

## Beschreibung

### Technisches Gebiet

Die Erfindung betrifft eine Verschlusskappe für ein Behältnis zur Aufnahme von medizinischen Flüssigkeiten, insbesondere zur Aufnahme von Flüssigkeiten, die im Bereich der Infusion, der Transfusion, der klinischen Ernährung oder der Dialyse eingesetzt werden. Des Weiteren betrifft die Erfindung ein Behältnis für medizinische Flüssigkeiten, das eine derartige Verschlusskappe aufweist.

### Stand der Technik

Die DE 10 2007 005 407 A1 offenbart eine Verschlusskappe für ein Behältnis zur Aufnahme von medizinischen Flüssigkeiten. Die Verschlusskappe weist einen Deckelteil und einen Randteil und einen in dem Deckelteil angeordneten ersten Anschluss und zweiten Anschluss auf. Der erste Anschluss ist als Zuspritzteil ausgebildet. Er umfasst ein nach außen weisendes Anschlussteil mit einer konischen Ausnehmung zur abdichtenden Aufnahme des Kegelschaftes einer nadellosen Injektionsspritze und einen nach innen weisenden Verschlussteil, der eine selbstabdichtende Membran zum Verschließen der Ausnehmung des Anschlussteils aufweist. Der zweite Anschluss ist als Entnahmeteil ausgebildet und umfasst einen nach außen weisenden Anschlussteil mit einer zylinderförmigen Ausnehmung und einen nach innen weisenden Verschlussteil, der eine selbstabdichtende Membran zum Verschließen der Ausnehmung des Anschlussteils aufweist. Die Verschlusskappe erlaubt über den ersten Anschluss das Zuspritzen einer Flüssigkeit, ohne dass eine Injektionsnadel verwendet werden muss, mittels des zweiten Anschlusses kann über einen Infusionsspike Flüssigkeit aus dem mit der Verschlusskappe verbundenen Behältnis entnommen werden.

### Kurzbeschreibung der Erfindung

Die erfindungsgemäße Verschlusskappe umfasst einen ersten Anschluss zum Zuspritzen einer medizinischen Flüssigkeit in ein mit der Verschlusskappe verbundenes Behältnis, insbesondere in ein Infusionsbehältnis, und einen zweiten Anschluss zum Entnehmen einer medizinischen Flüssigkeit aus einem mit der Verschlusskappe verbundenen Behältnis, insbesondere aus einem Infusionsbehältnis, wobei der erste Anschluss ein nach außen weisendes erstes Anschlussteil mit einer konischen Ausnehmung zur abdichtenden Aufnahme eines Kegelschaftes einer anzuschließenden ersten Vorrichtung und der zweite Anschluss ein nach außen weisendes zweites Anschlussteil mit einer konischen Ausnehmung zur abdichtenden Aufnahme eines Kegelschaftes einer zweiten anzuschließenden Vorrichtung aufweist. Dies ermöglicht es, eine Verschlusskappe mit mindestens zwei Anschlüssen auszubilden, die mit einer nadellosen oder infusionsspikelosen Vorrichtung jeweils dichtend verbunden werden können. Dadurch, dass Vorrichtungen ohne Nadel oder Infusionsspike mit beiden Anschlüssen verbunden werden können, wird die Gefahr einer Verletzung des Anwenders oder eine Beschädigung des Behältnisses, das mit der Verschlusskappe verbunden ist, erheblich reduziert. Zudem ist die Dichtheit einer derartigen Verbindung gewährleistet. Ist das erste/und oder zweite Anschlussteil mit einer Verrieglung ausgebildet, was erfindungsgemäß bevorzugt ist, kann zudem verhindert werden, dass sich durch beispielsweise ruckartige Bewegungen des Patienten oder durch Zugbelastung die Verbindung unbeabsichtigt löst mit der Folge, dass sich der Patient und/oder das Pflegepersonal mit hochtoxischen Medikamenten kontaminieren könnten und teure Medikamente wie Antibiotika oder Zytostatika möglicherweise verworfen werden müssten.

Grundsätzlich ist die Verschlusskappe für beliebige Behältnisse einsetzbar. Vorzugsweise ist die Verschlusskappe eine Verschlusskappe für ein Behältnis für die Aufnahme von medizinischen Flüssigkeiten, die in der Infusion, der Transfusion, der klinischen Ernährung oder der Dialyse verwendet werden. Vorzugsweise ist das Behältnis als Flasche ausgebildet, insbesondere als Kunststoffflasche. Bevorzugt ist die Kunststoffflasche als SBM(Stretch-Blow-Molding)-Behältnis ausgebildet.

In einer vorteilhaften Weiterbildung ist das erste und/oder das zweite Anschlussteil als Luer-Konnektor ausgebildet. Luer-Konnektoren sind Bestandteile von insbesondere in der Medizintechnik verwendeten normierten Kegelverbindungen mit einem Kegelschaft und einer Kegelhülse, die für den Anschluss medizinischer Vorrichtungen verwendet werden. Das erste und/oder das zweite Anschlussteil kann sowohl als nicht-verriegelbarer Luer-Konnektor oder als verriegelbarer Luer-Konnektor (auch "Luer-Lock-Konnektor" genannt) ausgebildet sein. Die Ausbildung des ersten und/oder zweiten Anschlussteils als Luer-Konnektor gewährleistet aufgrund der Normierung einen definierten Verbindungsmechanismus. Zudem ist eine Universalität der Anschlüsse gegeben, es entfällt der Umstand, für unterschiedliche Länder verschiedene Varianten von Anschlüssen bereitzuhalten und das Risiko eines Missbrauches durch einen Anschluss von nicht passenden Vorrichtungen ist reduziert. Vorzugsweise ist das erste und/oder das zweite Anschlussteil als Luer-Lock-Konnektor ausgebildet, was eine definierte Verriegelung der Verbindung mit einer anzuschließenden Vorrichtung ermöglicht und damit zur Sicherheit der Verbindung beiträgt, insbesondere gegen ein unbeabsichtigtes Lösen der Verbindung.

Gemäß einer weiteren vorteilhaften Weiterbildung umfasst der erste Anschluss eine erste Membran und ein erstes Durchstechelement, wobei die erste Membran zwischen der konischen Ausnehmung des ersten Anschlussteils und des Durchstechelementes angeordnet ist, so dass durch Anschließen der ersten Vorrichtung die erste Membran gegen das erste Durchstechelement gedrückt und durchstochen wird. Die Membran ermöglicht es, in ihrem geschlossenen Zustand einen Transport von Flüssigkeit durch den Anschluss zu verhindern und erst durch ein Öffnen einen Flüssigkeitstransport zu erlauben. Das Durchstechelement ermöglicht ein leichtes und definiertes Durchstechen der Membran und gewährleistet, dass die Membran tatsächlich geöffnet wird. Vorzugsweise ist die Membran derart ausgebildet und derart angeordnet, dass eine an den Anschluss anzuschließende Vorrichtung die Membran im Zuge des Anschließens öffnet. Beispielsweise kann die Vorrichtung einen Luer-Male-Anschluss mit einem Innenkegel umfassen sein, der die Membran beim Anschließen durchstößt und auf diese Weise öffnet. Das erste Anschlussteil ist entsprechend als Luer-Female-Anschluss oder Luer-Lock-Female-Anschluss ausgebildet, was erfindungsgemäß bevorzugt ist. Des Weiteren ist die Membran vorzugsweise als elastischer Körper ausgebildet, so dass die Membran beim Lösen der Vorrichtung in ihre Ausgangsstellung zurückkehrt, in der der Anschluss durch die Membran flüssigkeitsdicht verschlossen ist.

Vorzugsweise ist das Durchstechelement als hohlförmiger Körper mit einer Spitze ausgebildet. Durch die Spitze kann die Membran durchstochen werden. Dadurch, dass das Durchstechelement als hohlförmiger Körper ausgebildet ist, kann der Flüssigkeitstransport durch das Durchstechelement hindurch erfolgen. Die Spitze kann geeignet geschärft sein, um das Durchstechen der Membran zu erleichtern.

In einer weiteren vorteilhaften Ausbildung umfasst der zweite Anschluss entsprechend dem ersten Anschluss eine Membran und ein Durchstechelement. Das zweite Anschlussteil ist vorzugsweise als Luer-Female-Anschluss oder Luer-Lock-Female-Anschluss ausgebildet. Insbesondere kann für den ersten Anschluss und den zweiten Anschluss die gleiche Membran und/oder das gleiche Durchstechelement eingesetzt werden, wodurch die Anzahl unterschiedlicher Teile reduziert ist und damit Herstellungskosten gesenkt werden können. Des Weiteren können erster Anschluss und zweiter Anschluss identisch ausgebildet sein. Dies hat den Vorteil, dass in zeitlich kritischen Situationen, wie sie beispielsweise im Rettungsdienst oder im Operationssaal vorkommen können, die Fehlerrate erheblich minimiert werden kann, da es nicht mehr notwendig ist darauf zu achten, welcher Anschluss zum Entnehmen und welcher Anschluss zum Zuspritzen vorgesehen ist.

Das erfindungsgemäße Behältnis für medizinische Flüssigkeiten umfasst eine erfindungsgemäße Verschlusskappe. Vorzugsweise ist das Behältnis als Flasche ausgebildet.

Die abhängigen Ansprüche beschreiben weitere vorteilhafte Weiterbildungen der Erfindung.

Im Folgenden wird die Erfindung anhand von Ausführungsbeispielen, die durch mehrere Figuren dargestellt sind, näher erläutert.

### Kurze Beschreibung der Zeichnungsfiguren

Es zeigt:

Fig. 1 eine perspektivische Ansicht auf eine Ausführungsform einer erfindungsgemäßen Verschlusskappe,

Fig. 2 eine Unteransicht der in Fig. 1 gezeigten Verschlusskappe,

Fig. 3 eine Seitenansicht der in Fig. 1 gezeigten Verschlusskappe,

Fig. 4 eine Draufsicht der in Fig. 1 gezeigten Verschlusskappe,

Fig. 5 einen Querschnitt durch die gezeigte Verschlusskappe entlang der in der Fig. 4 eingezeichneten Schnittlinie A-A,

Fig. 6 eine perspektivische Ansicht der in Fig. 1 gezeigten Verschlusskappe mit einer an dem ersten Anschluss angeschlossenen Spritze,

Fig. 7 einen Querschnitt durch die in Fig. 6 gezeigte Verschlusskappe mit angeschlossener Spritze entlang einer Schnittlinie entsprechend der in der Fig. 4 eingezeichneten Schnittlinie A-A,

Fig. 8 eine perspektivische Ansicht der in Fig. 1 gezeigten Verschlusskappe mit einem an dem ersten Anschluss angeschlossenen Schlauch,

Fig. 9 einen Querschnitt durch die in Fig. 6 gezeigte Verschlusskappe mit angeschlossenem Überleitschlauch entlang einer Schnittlinie entsprechend der in der Fig. 4 eingezeichneten Schnittlinie A-A.

### Beschreibung der Ausführungsarten

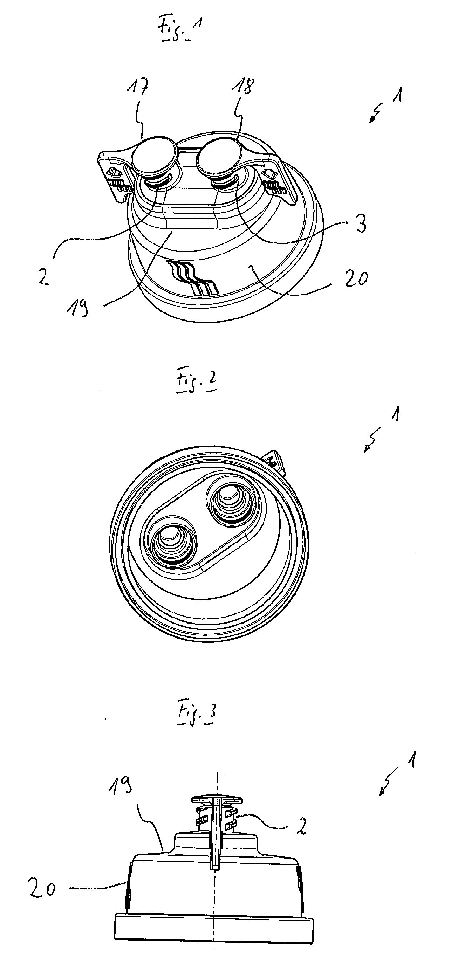

Die Figuren 1 bis 8 zeigen eine Ausführungsform einer erfindungsgemäßen Verschlusskappe 1 in verschiedenen Ansichten und mit verschiedenen angeschlossenen Vorrichtungen.

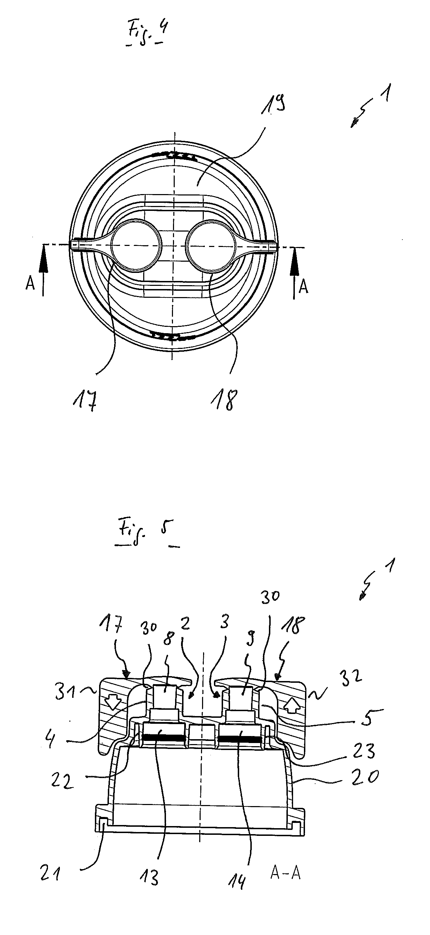

Die Verschlusskappe 1 ist als Verschlusskappe für ein Behältnis von medizinischen Flüssigkeiten ausgebildet und umfasst einen ersten Anschluss 2 zum Zuspritzen einer medizinischen Flüssigkeit und einen zweiten Anschluss 3 zum Entnehmen einer medizinischen Flüssigkeit. Der erste Anschluss 2 und der zweite Anschluss 3 sind in einem Deckelbereich 19 der Verschlusskappe 1 angeordnet. Des Weiteren umfasst die Verschlusskappe 1 einen Randbereich 20, der den Deckelbereich 19 umgibt, und der zum Aufsetzen der Verschlusskappe 1 auf ein Behältnis ausgebildet ist. In diesem Ausführungsbeispiel ist der Randbereich 20 derart ausgebildet, dass mittels diesem die Verschlusskappe 1 auf eine nicht näher dargestellte SBM-Kunststoffflasche aufsetzbar und mit dieser flüssigkeitsdicht und keimdicht/bakteriendicht verbindbar ist. Zu diesem Zwecke umfasst der Randbereich eine umlaufende Nut 21, siehe Fig. 5, mit der ein korrespondierender Steg oder Rand eines Anschlusses der Flasche in Eingriff gebracht werden kann. Die flüssigkeitsdichte und keimdichte Verbindung des Behältnisses mit der Verschlusskappe 1 kann auf verschiedene Weise erfolgen, beispielsweise durch Schweißen, Stecken, Schnappen, Schrauben, Umspritzen und/oder Kleben.

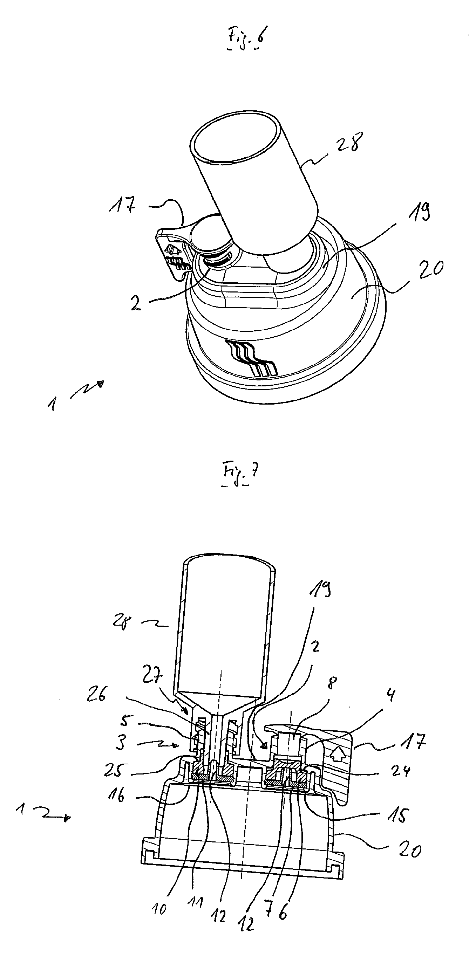

Der erste Anschluss 2 umfasst, siehe Fig. 5 und Fig. 7, ein erstes nach außen weisendes, d.h., vom anzuschließenden Behältnis weg weisendes, Anschlussteil 4 mit einer konischen Ausnehmung 8 zur abdichtenden Aufnahme eines Kegelschaftes einer anzuschließenden ersten Vorrichtung und ein zweites nach außen weisendes Anschlussteil 5 mit einer konischen Ausnehmung 9 zur abdichtenden Aufnahme eines Kegelschaftes einer zweiten anzuschließenden Vorrichtung. In diesem Ausführungsbeispiel ist sowohl das erste Anschlussteil 4 als auch das zweite Anschlussteil 5 als Luer-Konnektor ausgebildet, hier als Luer-Lock-Female-Konnektor gemäß ISO-Norm 594-2:1998 bzw. EN 1707:1996.

Des Weiteren umfasst der erste Anschluss 2 ein erstes Verschlussteil 22 mit einer ersten Aufnahme 13, der sich fluchtend an den ersten Anschlussteil 4 anschließt und nach innen weist, d.h., dem anzuschließenden Behältnis zugewandt ist, und der zweite Anschluss 3 ein zweites Verschlussteil 23 mit einer zweiten Aufnahme 14, der sich ebenfalls fluchtend an den ersten Anschlussteil 4 anschließt und nach innen weist. Die erste Aufnahme 13 und die zweite Aufnahme 14 sind in diesem Ausführungsbeispiel im Wesentlichen zylinderförmig ausgebildet. Das erste Verschlussteil 22 und das zweite Verschlussteil 23 liegen auf der dem anzuschließenden Behältnis zugewandten Seite des Deckelbereiches 19, das erste Anschlussteil 4 und das zweite Anschlussteil 5 auf der dem anzuschließenden Behältnis abgewandten Seite des Deckelbereiches 19.

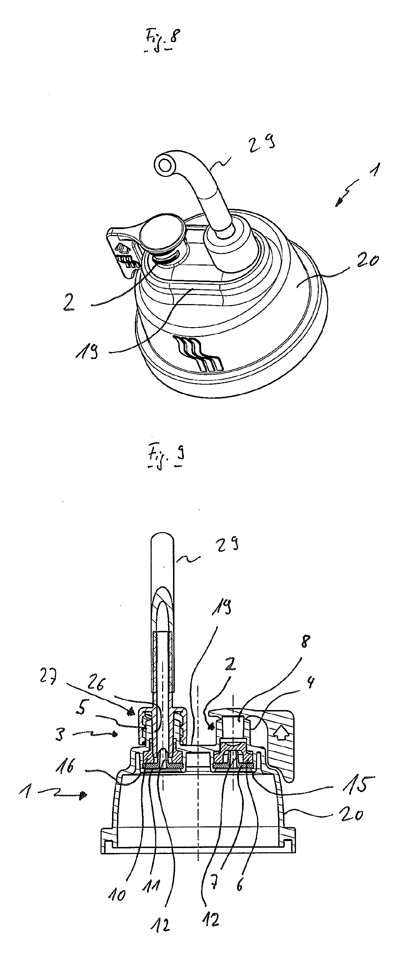

In der ersten Aufnahme 13 sind eine erste Membran 6 und ein erstes Durchstechelement 7 angeordnet, in der zweiten Aufnahme 14 sind eine zweite Membran 10 und ein zweites Durchstechelement 11 angeordnet siehe hierzu Fig. 7 oder Fig. 9 (in Fig. 5 wurden die Membranen 6, 10 und die Durchstechelemente 7, 11 der Übersicht wegen weggelassen). Die erste Membran 6 ist zwischen der konischen Ausnehmung 8 des ersten Anschlussteils 4 und des ersten Durchstechelementes 7 angeordnet, die zweite Membran 10 ist zwischen der konischen Ausnehmung 9 des zweiten Anschlussteils 5 und des zweiten Durchstechelementes 11 angeordnet. Die Membranen 6, 10 verhindern im geschlossenen Zustand einen Flüssigkeitstransport durch die beiden Anschlüsse 2, 3, im geöffneten Zustand der Membranen 6, 10 ist der Flüssigkeitstransport durch die Anschlüsse 2, 3 freigegeben. Die Membranen 6, 7 sind aus einem elastischen Material, so dass die Membranen aus einer geöffneten Stellung selbsttätig in die geschlossene Stellung übergehen und damit die Anschlüsse flüssigkeits- und keimdicht verschließen können. Die Figuren 7 und 9 zeigen die erste Membran 6 in einem geschlossenen Zustand, also in einem Zustand, in dem der Flüssigkeitstransport durch den ersten Anschluss 2 unterbunden ist, und die zweite Membran 10 in einem geöffnetem Zustand, bei dem der Flüssigkeitstransport durch den zweiten Anschluss 3 gewährleistet ist.

Das erste Durchstechelement 7 und das zweite Durchstechelemente 11 sind jeweils als steife, scheibenförmige Körper mit einer in Zentrum angeordneten, hohlen Spitze 12 ausgebildet. Die hohle Spitze gewährleistet den Transport einer Flüssigkeit durch das Durchstechelement 7, 11 hindurch. Die Spitzen 12 sind unterhalb den zugeordneten Membranen 6, 10 angeordnet und gegen die jeweilige Membran 6 bzw. 10 ausgerichtet, so dass bei einem anschlussseitigen Druck die jeweilige Membran 6 bzw. 10 gegen das Durchstechelement 7, 11 gepresst und auf diese Weise geöffnet wird. Um das Öffnen zu erleichtern, sind die Spitzen 12 der Durchstechelemente 7, 11 angeschärft. Als weitere Maßnahme, um das Öffnen zu erleichtern, sind die erste Membran 6 und die zweite Membran 11 im Zentrum geschlitzt.

Der erste Anschluss weist im Bereich des ersten Verschlussteils 22 eine nach innen gerichtete umlaufende Umbördelung 15 auf, durch die das erste Durchstechelement 7 formschlüssig hintergriffen und gegen die erste Membran 6 gepresst wird, so dass die erste Membran 6 formschlüssig zwischen dem ersten Durchstechelement 7 und einer vom ersten Verschlussteil 22 gebildeten Stufe 24 eingeklemmt wird. Dabei dichtet die Membran 6, unterstützt durch den vom ersten Durchstechelement 7 ausgeübten Anpressdruck, gegen die Innenwand des Verschlussteils 22 ab, so dass keine Flüssigkeit oder Keime zwischen Membran 6 und Innenwand des Verschlussteiles 22 unerwünscht durch den ersten Anschluss 2 gelangen können. Damit ist gewährleistet, dass nur bei geöffneter Membran 6 ein Flüssigkeitstransport erfolgt. Entsprechend weist der zweite Anschluss im Bereich des zweiten Verschlussteils 23 eine nach innen gerichtete umlaufende Umbördelung 16 auf, durch die das zweite Durchstechelement 11 formschlüssig hintergriffen und gegen die zweite Membran 10 gepresst wird, so dass die zweite Membran 10 formschlüssig zwischen dem zweiten Durchstechelement 11 und einer vom zweiten Verschlussteil 23 gebildeten Stufe 25 eingeklemmt wird. Die zweite Membran 10, unterstützt durch den vom zweiten Durchstechelement 11 ausgeübten Anpressdruck, gegen die Innenwand des zweiten Verschlussteils 23 ab, so dass keine Flüssigkeit zwischen Membran 10 und Innenwand des Verschlussteiles 23 unerwünscht durch den zweiten Anschluss 3 gelangen kann.

Die Figur 5 zeigt die Verschlusskappe 1 ohne die Umbördelungen 15, 16. Anschlüsse 2, 3, Deckelbereich 19 und Randbereich 20 weisen keinen Hinterschnitte auf, was einen einfache und kostengünstige Herstellung der Verschlusskappe 1 ermöglicht, da aufwendige Mechanismen zum Entformen entfallen. Das Umbördeln der Ränder der Verschlussteile 22, 23 erfolgt nach einem Einsetzen der Membrane 6, 10 und der Durchstechelemente 7, 11, wodurch man die in den Figuren 7, 9 gezeigte Verschlusskappe 1 erhält.

Der erste Anschluss 2 und der zweite Anschluss 3 sind derart ausgebildet, dass durch das Anschließen einer Vorrichtung mit einem Luer-Lock-Male-Konnektor im Zuge des Verriegelns der Vorrichtung mit dem jeweiligen Anschluss der hohle Innenkegel des Luer-Lock-Male-Konnektors die jeweilige Membran 6, 10 des Anschlusses 2, 3 gegen das Durchstechelement 7, 11 presst, wodurch die Membran 6, 10 geöffnet wird. Die aufzuwendende Kraft zum Durchstechen der Einstechstelle ist im Vergleich zu einer herkömmlichen Verbindung mit einem Infusionsspike sehr gering. Dadurch ist diese Verbindung anwenderfreundlich, sicher, zuverlässig und berührungsgeschützt.

Der Außendurchmesser der hohlen Spitze 12 ist an den Innendurchmesser des Innenkegels des Luer-Lock-Male-Konnektors angepasst, so dass sich der Innenkegel über einen Abschnitt der Spitze 12 schieben kann. Dies ist exemplarisch durch die Figuren 7, 9 dargestellt, wo eine nadellose Spritze 28 bzw. ein Überleitschlauch 29 mit einem Luer-Lock-Male-Konnektor 27, der einen hohlförmigen Innenkegel 26 umfasst, an den zweiten Anschluss 3 angeschlossen sind. Nach Anschluss der jeweiligen Vorrichtung 28, 29 erfolgt der Flüssigkeitstransport durch den Innenkegel 26 und die Spitze 12 des Durchstechelementes 11. Entfernt man die angeschlossene Vorrichtung, so kehren aufgrund ihrer Elastizität und Rückstellkraft die Membrane 6, 10 in ihren Ausgangszustand zurück und verschließen die Anschlüsse 2, 3 wieder flüssigkeitsdicht.

Der erste Anschluss 2 und der zweite Anschluss 3 sind bis auf das Außengewinde des Luer-Locks im Wesentlichen rotationssymmetrisch ausgebildet. Des Weiteren sind in diesem Ausführungsbeispiel Anschluss 2 und Anschluss 3 einschließlich der Membranen 6, 10 und Durchstechelemente 7, 11 identisch.

Der erste Anschluss 2 ist mit einem Abbrechteil 17 flüssigkeitsdicht und bakteriendicht verschlossen, der zweite Anschluss 3 mit einem zweiten Abbrechteil 18 flüssigkeitsdicht und bakteriendicht verschlossen. Die Abbrechteile 17, 18 verhindern eine Verschmutzung der Anschlüsse 2, 3 vor deren Benutzung und gewährleisten eine Sterilität und Unversehrtheit des Innenraums. Die Abbrechteile 17, 18 sind über eine Sollbruchstelle 30, siehe Fig. 5, mit dem jeweiligen Anschluss 2, 3 verbunden und weisen jeweils eine Lasche 31, 32 auf, über dies die Abbrechteile 17, 18 manuell von dem jeweiligen Anschluss abreißbar sind. Die Abbrechteile 17, 18 können mit Zeichen versehen sein, die eine Flüssigkeitstransportrichtung anzeigen, beispielsweise mit Pfeilen als Ausnehmung oder als Prägung. Unabhängig von solchen Zeichen sind in diesem Ausführungsbeispiels die Anschlüsse 2, 3 für einen Flüssigkeitstransport in beide Richtungen ausgebildet.

Die Anschlüsse 2, 3, Abbrechteile 17, 18, Deckelbereich 19 und Randbereich 20 sind hier als einteiliges, insbesondere einstückiges Spritzgussteil aus einem Kunststoff ausgebildet. Anschlüsse 2, 3, Deckelbereich 19, Randbereich 20 und Abbrechteile 17, 18 sind in diesem Falle ein Polypropylen. Alternativ wäre beispielsweise auch Polyethylen (PE) oder HDPE (High Density PE) geeignet. Das jeweilige Durchstechelement 7, 11 ist ebenfalls ein Spritzgussteil aus Kunststoff, beispielsweise aus Polypropylen (PP) oder Polycarbonat (PC) oder Acrylnitril-Butadien-Styrol (ABS). Alternativ kann das Durchstechelement 7, 11 auch aus Metall sein. Dies ist insbesondere für den Fall vorteilhaft, dass auf eine Schlitzung der Membrane 6, 10 verzichtet werden soll. Die jeweilige Membran 6, 10 ist eine Membran aus einem elastischen Material, beispielsweise aus einem thermoplastischen Elastomer (TPE) oder aus Polyisoprenen. Alternativ kann die Membran auch aus einem Silikon, Chlor- oder Brombuthyl bestehen.

In dem Ausführungsbeispiel wurde eine Verschlusskappe 1 mit zwei identischen Anschlüsse 2, 3 beschrieben. Obwohl dies eine bevorzugte Ausführungsform ist, ist es selbstverständlich möglich, die Anschlüsse 2, 3 verschieden voneinander auszubilden. Als eine weitere Alternative kann eines der Anschlussteile 4, 5 oder können beide Anschlussteile 4, 5 als ein Luer-Konnektor ohne Lock-Funktion, insbesondere als Luer-Female-Konnektor, ausgebildet werden. Des Weiteren kann das erste und/oder das zweite Anschlussteil 4, 5 alternativ auch als ein luer-ähnlicher Konnektor ausgebildet sein, beispielsweise als ein Large-Bore-Konnektor oder als ein Super-Large-Bore-Konnektor. Ein Large-Bore-Konnektor oder Super-Large-Bore-Konnektor ermöglicht eine höhere Flussrate als ein Luer-Konnektor, was beispielsweise vorteilhaft für die Dialyse oder für die Übertragung einer Spüllösung in der Urologie oder Arthroskopie ist.

## Patentansprüche

1. Verschlusskappe (1) für ein Behältnis zur Aufnahme von medizinischen Flüssigkeiten, umfassend einen ersten Anschluss (2) zum Zuspritzen einer medizinischen Flüssigkeit und einen zweiten Anschluss (3) zum Entnehmen einer medizinischen Flüssigkeit, wobei der erste Anschluss (2) ein nach außen weisendes erstes Anschlussteil (4) mit einer konischen Ausnehmung (8) zur abdichtenden Aufnahme eines Kegelschaftes (26) einer anzuschließenden ersten Vorrichtung (28, 29) und der zweite Anschluss (3) ein nach außen weisendes zweites Anschlussteil (5) mit einer konischen Ausnehmung (9) zur abdichtenden Aufnahme eines Kegelschaftes einer anzuschließenden zweiten Vorrichtung aufweist.

2. Verschlusskappe (1) nach Anspruch 1, wobei das erste Anschlussteil (4) und/oder das zweite Anschlussteil (5) als Luer-Konnektor ausgebildet sind, vorzugsweise als Luer-Lock-Female-Konnektor ausgebildet sind.

3. Verschlusskappe (1) nach einem der vorhergehenden Ansprüche, wobei der erste Anschluss (2) eine erste Membran (6) und ein erstes Durchstechelement (7) aufweist, wobei die erste Membran (6) zwischen der konischen Ausnehmung (8) des ersten Anschlussteils (4) und des Durchstechelementes (7) angeordnet ist, so dass durch Anschließen der ersten Vorrichtung die erste Membran (6) gegen das erste Durchstechelement (7) gedrückt und vom ersten Durchstechelement (7) durchstochen wird.

4. Verschlusskappe (1) nach einem der vorhergehenden Ansprüche, wobei der zweite Anschluss (3) eine zweite Membran (10) und ein zweites Durchstechelement (11) aufweist, wobei die zweite Membran (10) zwischen der konischen Ausnehmung (9) des zweiten Anschlussteils (5) und des zweiten Durchstechelementes (11) angeordnet ist, so dass durch Anschließen der zweiten Vorrichtung die zweite Membran (10) gegen das zweite Durchstechelement (11) gedrückt und vom zweiten Durchstechelement (11) durchstochen wird.

5. Verschlusskappe (1) nach einem der Ansprüche 3 oder 4, wobei das erste Durchstechelement (7) als hohlförmiger Körper mit einer Spitze (12) ausgebildet ist und/oder das zweite Durchstechelement (11) als hohlförmiger Körper mit einer Spitze (12) ausgebildet ist.

6. Verschlusskappe (1) nach einem der vorhergehenden Ansprüche, wobei die erste Membran (6) und/oder die zweite Membran (10) geschlitzt sind.

7. Verschlusskappe (1) nach einem der vorhergehenden Ansprüche, wobei der erste Anschluss (2) eine erste Aufnahme (13) aufweist, in die erste Membran (6) und erstes Durchstechelement (7) eingesetzt sind und/oder der zweite Anschluss (3) eine zweite Aufnahme (14) aufweist, in die zweite Membran (10) und zweites Durchstechelement (11) eingesetzt sind.

8. Verschlusskappe (1) nach Anspruch 7, wobei der erste Anschluss (2) eine erste Umbördelung (15) aufweist, durch die erste Membran (6) und erstes Durchstechelement (7) formschlüssig in der ersten Aufnahme (13) befestigt sind und/oder der zweite Anschluss (3) eine zweite Umbördelung (16) aufweist, durch die zweite Membran (10) und zweites Durchstechelement (11) formschlüssig in der zweiten Aufnahme (14) befestigt sind.

9. Verschlusskappe (1) nach einem der vorhergehenden Ansprüche, zusätzlich umfassend ein erstes Abrechteil (17), das mit dem ersten Anschluss (2) verbunden ist und diesen flüssigkeitsdicht verschließt, und/oder ein zweites Abrechteil (18), das mit dem zweiten Anschluss (3) verbunden ist und diesen flüssigkeitsdicht verschließt.

10. Verschlusskappe (1) nach einem der vorhergehenden Ansprüche, zusätzlich umfassend einen Deckelbereich (19) und einen Randbereich (20), wobei der erste Anschluss (2) und der zweite Anschluss (3) in dem Deckelbereich (20) angeordnet ist.

11. Verschlusskappe (1) nach einem der vorhergehenden Ansprüche, wobei erster Anschluss (2), zweiter Anschluss (3) und gegebenenfalls Randbereich (19) und Deckelbereich (20) und gegebenenfalls erstes Abrechteil (17) und zweites Abrechteil (18) einteilig, vorzugsweise einstückig, ausgeführt sind.

12. Behältnis für medizinische Flüssigkeiten umfassend eine Verschlusskappe (1) nach einem der vorhergehenden Ansprüche.

13. Behältnis nach Anspruch 12, wobei das Behältnis als Flasche, vorzugsweise als SBM (Stretch-Blow-Molding)-Flasche ausgebildet ist.
